# NEW EUROPEAN PATENT SPECIFICATION

(11) **EP 2 723 308 B2**
(45) Date of publication and mention of the opposition decision: **30.08.2023**
(45) Mention of the grant of the patent: 02.08.2017
(21) Application number: 12732596.7
(22) Date of filing: 22.06.2012
(51) Int. Cl.: A61K 8/84, A61K 8/34, A61K 8/41, A61K 8/46, A61Q 5/06, A61K 8/97, A61Q 5/12, A61K 8/81, A61Q 5/02, A61K 8/891, A61K 8/92, A61K 8/36, A61Q 5/00, A61K 8/89

(54) **COSMETIC COMPOSITION COMPRISING AT LEAST ONE PARTICULAR AMPHOTERIC POLYMER AND AT LEAST ONE PARTICULAR CONDITIONING AGENT**
KOSMETISCHE ZUSAMMENSETZUNG MIT MINDESTENS EINEM SPEZIELLEN AMPHOTEREN POLYMER UND MINDESTENS EINEM SPEZIELLEN KONDITIONIERER
COMPOSITION COSMÉTIQUE COMPRENANT AU MOINS UN POLYMÈRE AMPHOTÈRE PARTICULIER ET AU MOINS UN AGENT DE CONDITIONNEMENT PARTICULIER

(30) Priority: 23.06.2011 FR 1155555; 23.06.2011 FR 1155584; 23.06.2011 FR 1155556; 29.08.2011 US 201161528615 P; 29.08.2011 US 201161528606 P; 29.08.2011 US 201161528715 P
(43) Date of publication of application: 30.04.2014
(73) Proprietor: L'OREAL, 75008 Paris (FR)
(72) Inventor: MERALLI, Sabina, F-94260 FRESNES (FR); FALLOU, Bénédicte, F-95220 Herblay (FR); LESCH, Sandie, F-75011 Paris (FR)
(74) Representative: Casalonga
(86) International application number: PCT/EP2012/062100
(87) International publication number: WO 2012/175682

(56) References cited:
- EP-A1- 0 522 756
- EP-A1- 1 064 917
- EP-A1- 1 366 738
- WO-A1-00/06097
- WO-A1-00/64410
- WO-A1-2005/099654
- WO-A2-2010/149460
- DE-U1-202008 013 432
- JP-A- 2010 215 531
- US-A- 5 344 643
- US-A1- 2005 129 648
- US-A1- 2005 276 778
- DATABASE gnpd [Online] mintel; September 2002 (2002-09), "Illipe Butter Conditioner", XP002671038, Database accession no. 10117732
- DATABASE gnpd [Online] Mintel; May 2009 (2009-05), "Bone Marrow Hair Treatment", XP002671039, Database accession no. 1086617
- DATABASE gnpd [Online] Mintel; October 2010 (2010-10), "Four-Week Hair Food Treatment", XP002671040, Database accession no. 1422117
- DATABASE gnpd [Online] Mintel; April 2011 (2011-04), "Continuous Hydrating Treatment", XP002671041, Database accession no. 1533578
- DATABASE gnpd [Online] mintel; April 2008 (2008-04), "Moisturizing Combing Cream", XP002672304, Database accession no. 898758
- DATABASE gnpd [Online] mintel; May 2011 (2011-05), "Continuous Straightening Treatment", XP002672308, Database accession no. 1533147
- ANONYMOUS: "Conditioning polymers provide multiple benefits", INTERNET CITATION, 1 March 2011 (2011-03-01), pages 1-3, XP002662657, Retrieved from the Internet: URL:http://www.personalcaremagazine.com/Pr int.aspx?Story=7937 [retrieved on 2011-10-21]
- "Lubrizol & Nalco Present Naturally Derived Polymers for skin & Hair Care at in-Cosmetic 2011", INTERNET CITATION, 30 March 2011 (2011-03-30), XP002669583, Retrieved from the Internet: URL:www.specialchem4cosmetics.com/services /news.aspx?id=6389 [retrieved on 2012-03-05]
- "Merquat 2001 polymer, Technical Data Sheet", LUBRIZOL, 23 February 2012 (2012-02-23),

## Description

The present invention relates to a cosmetic composition for treating keratin fibres, in particular human keratin fibres such as the hair, comprising one or more particular amphoteric polymers and one or more particular conditioning agents.

The present invention also relates to a cosmetic process for treating keratin fibres using such a composition and also to a cosmetic use of the said composition.

Thus, to improve the cosmetic properties of keratin fibre care compositions, for example of those that are required to be applied to sensitized hair (i.e. hair that is generally damaged or embrittled by the action of external atmospheric agents such as light and bad weather, and/or mechanical or chemical treatments such as blow-drying, combing, dyeing, bleaching, permanent-waving and/or relaxing), it is known practice to introduce into these compositions cosmetic agents known as conditioning agents, which are intended mainly to repair or limit the harmful or undesirable effects caused by the various treatments or attacking factors to which the hair fibres are more or less repeatedly subjected. These conditioning agents may, of course, also improve the cosmetic behaviour of natural hair.

Thus, users with dyed or damaged hair often resort to haircare treatments, and most particularly to treating shampoos combining a silicone and a cationic polymer. These treating shampoos have the advantage of washing the hair, just like a standard shampoo, while at the same time giving the hair conditioning properties, which are particularly sought for the treatment of sensitized hair. Furthermore, these products are frequently used by users and do not require an additional treatment step, which may prove to be burdensome or a constraint in certain cases.

These shampoos combining a silicone and a cationic polymer give a sensation of fluidity and lightness, especially due to the use of the silicones. However, following repeated use of this type of shampoo, a build-up effect may appear depending on the cationic polymer used and its concentration in the shampoo. This build-up effect is due to the successive deposition of the conditioning agent on the fibres, without the agent previously deposited having been correctly removed. Thus, the deposit of the conditioning agent is then more and more thick, leading to lankness of the fibres.

Users are thus seeking to obtain a fluid conditioning effect on keratin fibres, without any lankness effect in the course of the applications, and with a long-lasting sensation of cleanliness of the said fibres. A sensation of regularity of the cosmetic feel obtained, especially homogeneity of the feel from the root to the end of the keratin fibres, is also sought.

Finally, users are also in search of products that have a pleasant visual appearance and texture.

There is thus a need to develop cosmetic compositions for satisfactorily conditioning the hair, and especially for giving the hair good properties in terms of the lightness, smoothness, sheen, feel and disentangling of the hair, without making the fibres lank after repeated use of the composition.

Compositions are also sought which are stable over time, which have good working properties (which are especially easy to apply and to remove by rinsing and which, where appropriate, have good lathering properties), which are pleasant to use and which have an aesthetic visual appearance.

The Applicant has now discovered, surprisingly, that a combination of a particular amphoteric polymer and a particular conditioning agent makes it possible to achieve the objectives outlined above, especially on fibres that are sparingly to very sensitized.

Homogeneous cosmetic effects were obtained on the fibres, without any build-up effect by superposition. Good ease of styling of the head of hair was observed. Furthermore, on account of the lankness-free property of the head of hair, a satisfactory volume of the head of hair was obtained.

One subject of the present invention is thus a cosmetic composition comprising:
(a)- one or more amphoteric polymers comprising a repetition of:
   (i) one or more units obtained from a monomer of (meth)acrylamide type,
   (ii) one or more units obtained from a monomer of (meth)acrylamidoalkyltrialkylammonium type, and
   (iii) one or more units obtained from an acidic monomer of (meth)acrylic acid type,
   the said amphoteric polymer(s) being present in the composition in an amount of between 0.01% and 10% by weight relative to the total weight of the composition, and
(b)- one or more conditioning agents chosen from plant oils, mineral oils, fatty acid diesters of polyethylene glycol comprising from 2 to 50 oxyethylene units, and a mixture thereof,
the composition may further comprise additional conditioning agents chosen from non-volatile linear silicones of polydialkylsiloxane structure, non-volatile linear silicones of polydiarylsiloxane structure, and non-volatile linear silicones of polyalkylarylsiloxane structure;
the total amount of said conditioning agent(s) present in the composition being between 0.01% and 20% by weight, relative to the total weight of the composition.

This composition makes it possible especially to obtain homogeneous cosmetic effects on the fibres, without any "build-up" effect or any lankness of the head of hair. The hair, including fibres that are sparingly to very sensitized, are soft, shiny and supple, with a long-lasting sensation of cleanliness, and are easy to style. This composition also makes it possible to obtain a satisfactory volume of the head of hair and, for curly hair, good curl definition.

Furthermore, the composition according to the present invention has a particularly aesthetic nacreous appearance and a particularly pleasant texture when the fatty acid diester of polyethylene glycol is used as conditioning agent.

The present invention also relates to a cosmetic process for treating keratin materials, especially human keratin fibres such as the hair, which consists in applying to the keratin materials a composition according to the invention.

In particular, the composition according to the invention may be rinsed out or left on, and applied with or without the effect of heat.

Preferably, the compositions according to the invention are used as shampoo for washing and conditioning the hair, or as haircare products.

A subject of the present invention is also the use of the composition according to the invention for facilitating the styling and/or for improving the suppleness, smoothness and/or disentangling of the hair.

It should be noted that the compositions according to the invention are also stable on storage, both at room temperature (20-25°C) and at 45°C, especially as regards their visual aspect and their viscosity.

For the purposes of the present invention, the term "stable" means that the visual aspect and the viscosity of these compositions do not change substantially over time under storage test conditions, for example at room temperature (20°C-25°C) and/or at 45°C and/or at 4°C for two months following their manufacture.

Other subjects, characteristics, aspects and advantages of the invention will become even more clearly apparent on reading the description and examples which follow.

In the text hereinbelow, unless otherwise indicated, the limits of a range of values are included in that range, especially in the expressions "between" and "ranging from ... to ...".

In the text hereinbelow, the term "at least one" is equivalent to "one or more".

### Amphoteric polymer

The composition according to the present invention contains one or more amphoteric polymers comprising a repetition of:
(i) one or more units obtained from a monomer of (meth)acrylamide type,
(ii) one or more units obtained from a monomer of (meth)acrylamidoalkyltrialkylammonium type, and
(iii) one or more units obtained from an acidic monomer of (meth)acrylic acid type.

Preferably, the units derived from a monomer of (meth)acrylamide type (i) of the amphoteric polymer are units of structure (I) below: 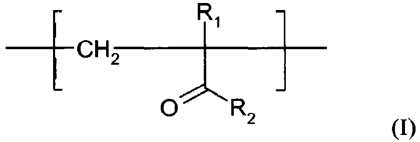 in which:
- R₁ denotes H or CH₃,
- R₂ is chosen from an amino, dimethylamino, tert-butylamino or dodecylamino radical, or -NH-CH₂OH.

Preferably, the amphoteric polymer of the invention comprises a repetition of only one unit of formula (I).

The unit derived from a monomer of (meth)acrylamide type of formula (I) in which R₁ denotes H and R₂ is an amino radical is particularly preferred. It corresponds to the acrylamide monomer per se.

Preferably also, the units derived from a monomer of (meth)acrylamidoalkyltrialkylammonium type (ii) of the amphoteric polymer are units of structure (II) below: 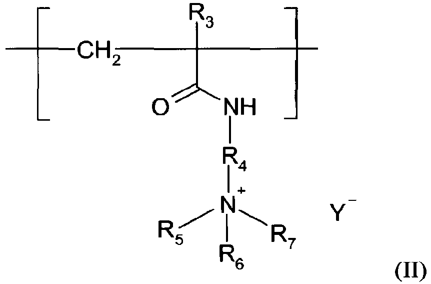 in which:
- R₃ denotes H or CH₃,
- R₄ denotes a group (CH₂)ₖ with k being an integer ranging from 1 to 6 and preferably from 2 to 4;
- R₅ and R₆, and R₇, which may be identical or different, each denote an alkyl group containing from 1 to 4 carbon atoms;
- Y⁻ is an anion such as bromide, chloride, acetate, borate, citrate, tartrate, bisulfate, bisulfite, sulfate or phosphate.

Among these units derived from a monomer of (meth)acrylamidoalkyltrialkylammonium the ones that are preferred are those derived from the methacrylamidopropyltrimethylammonium chloride monomer, which R₃ denotes a methyl radical, k is equal to 3, R₅, R₆ and R₇ denote a methyl radical, and Y⁻ denotes a chloride anion.

Preferably, the amphoteric polymer of the invention comprises a repetition of only one unit of formula (II).

Finally, the units derived from an acidic monomer of (meth)acrylic acid type (iii) of the amphoteric polymer are preferentially chosen from the units of formula (III): 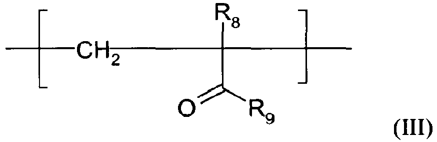 in which:
- R₈ denotes H or CH₃,
- R₉ denotes a hydroxyl radical or a radical -NH-C(CH₃)₂-CH₂-SO₃H.

The preferred units of formula (III) correspond to the acrylic acid, methacrylic acid and 2-acrylamino-2-methylpropanesulfonic acid monomers.

Preferably, the unit derived from an acidic monomer of (meth)acrylic acid type is that derived from acrylic acid, for which R₈ denotes a hydrogen atom and R₉ denotes a hydroxyl radical.

The acidic monomer(s) of (meth)acrylic acid type may be non-neutralized or partially or totally neutralized with an organic or mineral base.

Preferably, the amphoteric polymer of the invention comprises a repetition of only one unit of formula (III).

According to one preferred embodiment of the invention, the amphoteric polymer(s) comprise at least 30 mol% of units derived from a monomer of (meth)acrylamide type. Preferably, they comprise from 30 mol% to 70 mol% and more preferably from 40 mol% to 60 mol% of units derived from a monomer of (meth)acrylamide type.

The contents of units derived from a monomer of (meth)acrylamidoalkyltrialkylammonium type may advantageously be the following: from 10 mol% to 60 mol% and preferentially from 20 mol% to 55 mol%.

The contents of units derived from an acidic monomer of (meth)acrylic acid type may advantageously be the following: from 1 mol% to 20 mol% and preferentially from 5 mol% to 15 mol%.

According to one preferred embodiment of the invention, the amphoteric polymer comprises:
- from 30 mol% to 70 mol% and more preferably from 40 mol% to 60 mol% of units derived from a monomer of (meth)acrylamide type,
- from 10 mol% to 60 mol% and preferentially from 20 mol% to 55 mol% of units derived from a monomer of (meth)acry lamidoalkyltrialkylammonium type;
- from 1 mol% to 20 mol% and preferentially from 5 mol% to 15 mol% of units derived from an acidic monomer of (meth)acrylic acid type.

The amphoteric polymer(s) according to the present invention may also comprise additional units, other than the units derived from a monomer of (meth)acrylamide type, of (meth)acrylamidoalkyltrialkylammonium type and of (meth)acrylic acid type.

According to one preferred embodiment of the invention, the amphoteric polymer(s) consist solely of units derived from monomers (i) of (meth)acrylamide type, (ii) of (meth)acrylamidoalkyltrialkylammonium type and (iii) of (meth)acrylic acid type.

As examples of amphoteric polymers that are particularly preferred, mention may be made of acrylamide/methylacrylamidopropyltrimethylammonium chloride/acrylic acid terpolymers. Such polymers are listed in the CTFA dictionary. International Cosmetic Ingredient Dictionary, 10th edition 2004, under the name Polyquaternium 53. Corresponding products are especially sold under the names Merquat 2003 or Merquat 2003PR by the company Nalco.

The amphoteric polymer according to the invention may conventionally be prepared by polymerization starting with its various monomers, according to techniques known to those skilled in the art and especially by radical polymerization.

The amphoteric polymer(s) (a) are present in the composition according to the invention in an amount of between 0.01% and 10% by weight, preferably between 0.02% and 10% by weight, and preferably between 0.05% and 5% by weight, relative to the total weight of the composition.

### Conditioning agents

The composition according to the present invention also comprises one or more conditioning agents chosen from plant oils, mineral oils, fatty acid diesters of polyethylene glycol comprising from 2 to 50 oxyethylene units, and a mixture thereof.

The composition may further comprise additional conditioning agents chosen from non-volatile linear silicones of polydialkylsiloxane structure, non-volatile linear silicones of polydiarylsiloxane structure, and non-volatile linear silicones of polyalkylarylsiloxane structure.

### Plant or mineral oils

The composition according to the present invention may comprise one or more plant oils and/or mineral oils, as conditioning agent.

The term "oil" means any nonionic lipophilic compound that is insoluble in water and liquid at room temperature (25°C) and at atmospheric pressure. For the purposes of the present invention, the term "water-insoluble" refers to a compound whose solubility at spontaneous pH in water at 25°C and at atmospheric pressure is less than 1% and preferably less than 0.5%. The oils preferably have a melting point of less than 5°C and a viscosity of less than 500 cPs at 25°C at a shear rate of 1 s⁻¹.

In particular, the term "plant oil" means a cosmetically acceptable oil as defined above, obtained from a species belonging to the plant kingdom.

The plant oils used in the present invention are chosen from the plant oils usually used in the cosmetics field.

As examples of plant oils that may be used in the compositions of the invention, mention may be made of:
- sweet almond oil,
- apricot kernel oil,
- argan oil,
- babassu oil,
- avocado oil,
- groundnut oil,
- candlenut oil,
- camellia oil,
- camelina oil,
- safflower oil,
- beauty-leaf oil,
- rapeseed oil,
- coconut oil,
- coriander oil,
- marrow oil,
- wheatgerm oil,
- jojoba oil,
- linseed oil,
- macadamia oil,
- corn germ oil,
- hazelnut oil,
- walnut oil,
- vernonia oil,
- olive oil,
- evening primrose oil,
- palm oil,
- passion flower oil,
- grapeseed oil,
- pracaxi oil,
- rose oil,
- castor oil,
- rye oil,
- sesame oil,
- rice bran oil,
- soybean oil,
- tamanu oil, and
- sunflower oil.

The plant oils according to the invention have not undergone a chemical transformation after extraction, with the exception of a possible hydrogenation.

Among the plant oils mentioned above, use is preferably made of olive oil, argan oil, avocado oil, rapeseed oil, jojoba oil, soybean oil, apricot kernel oil and sunflower oil, and more preferably olive oil, apricot kernel oil and avocado oil.

Essential oils may also be used. Essential oils differ from plant oils by the fact that they cannot be decomposed by saponification into glycerol and fatty acid soap. Moreover, they are volatile.

According to the definition given in international standard ISO 9235 and adopted by the Commission of the European Pharmacopoeia, an essential oil is a product generally of complex composition, obtained from a botanically defined plant raw material, either by steam entrainment, or by dry distillation, or by extraction using liquid or gaseous solvents, or via an appropriate mechanical process without heating (cold pressing). The essential oil is usually separated from the aqueous phase via a physical process that does not result in any significant change in the composition. These essential oils may also be prepared by synthesis.

The essential oil may be chosen from essential oil of cinnamon, essential oil of ginger, essential oil of black pepper, essential oil of pimento leaf, essential oil of peppermint and essential oil of clove, and mixtures thereof.

The term "mineral oils" means hydrocarbons in the form of linear or branched, saturated or unsaturated oils, of mineral or synthetic origin, and which may be hydrogenated.

The mineral oils used in the present invention are chosen from the mineral oils as defined above, usually used in the cosmetics field.

As examples of mineral oils that may be used in the present invention, mention may be made of:
- mixtures of hydrocarbon-based oils derived from petroleum (INCI name: Mineral Oil),
- volatile or non-volatile liquid paraffin,
- liquid petroleum jelly,
- polyolefins and in particular polydecenes,
- isoparaffins such as isohexadecane, isododecane and hydrogenated polyisobutylenes such as Parleam^{®} oil sold by the company NOF Corporation (INCI name: Hydrogenated polyisobutene).

Among the mineral oils mentioned above, the following are preferably used:
- mixtures of hydrocarbon-based oils derived from petroleum,
- volatile or non-volatile liquid paraffin, and
- liquid petroleum jelly, and
- polyolefins and in particular polydecenes.

The term "polydecenes" means any compound of formula C₁₀ₙH₍₂₀ₙ₎₊₂ in which n ranges from 3 to 9, corresponding to the name "polydecene" in the CTFA dictionary, 7th edition, 1997 of the Cosmetic, Toiletry and Fragrance Association, USA, and also to the same INCI name in the USA and in Europe. These are poly-1-decene hydrogenation products. Among these compounds, those for which, in the formula, n ranges from 3 to 7 are more particularly chosen according to the invention.

Examples that may be mentioned include the products sold under the name Silkflo^{®} 366 NF Polydecene by the company Amoco Chemical, and those sold under the names Nexbase^{®} 2002 FG, 2004 FG, 2006 FG and 2008 FG by the company Fortum.

The preferred mineral oil is liquid petroleum jelly.

In one preferred embodiment of the invention, mineral oil is liquid petroleum jelly and the non-volatile plant oil is chosen from olive oil, argan oil, avocado oil, apricot kernel oil, rapeseed oil, jojoba oil, soybean oil and sunflower oil.

The total amount of mineral oil(s) and plant oil(s), when they are present in the final composition, preferably ranges from 0.01% to 20% by weight, better still from 0.1% to 10% by weight and more particularly from 0.5% to 5% by weight relative to the total weight of the final composition.

Preferably, the weight ratio of the amount of amphoteric polymer(s) to the amount of plant oils and/or mineral oils is less than or equal to 10, better still less than or equal to 5 and even better still less than 1.

Even more preferentially, this ratio ranges from 0.01 to 10, better still from 0.05 to 5 and even better still from 0.1 to 2.

### Non-volatile linear silicone

The composition according to the present invention may also contain one or more non-volatile linear silicones chosen from polydialkylsiloxanes, polydiarylsiloxanes and polyalkylarylsiloxanes.

In the context of the invention, the term "non-volatile linear silicone" means a silicone with a viscosity of greater than or equal to 5 cSt at 25°C, especially a silicone oil, with a vapour pressure of less than 0.1 mmHg at 25°C. According to one particular embodiment, this viscosity is between 5 cSt and 1 000 000 cSt, preferably between 5 cSt and 100 000 cSt and even more preferentially from 100 to 10 000 cSt.

The non-volatile linear silicone(s) may be chosen especially from the silicones of formula (IV): 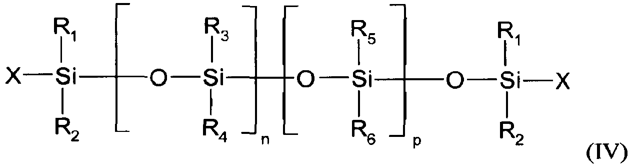 in which:
R₁, R₂, R₅ and R₆ denote, together or separately, an alkyl radical containing 1 to 6 carbon atoms,
R₃ and R₄ denote, together or separately, an alkyl radical containing from 1 to 6 carbon atoms or an aryl radical,
X is an alkyl radical containing from 1 to 6 carbon atoms or a hydroxyl radical,
n and p are integers selected such as to give a compound with a viscosity of more than 5 cSt; the sum n + p is preferably greater than 10.

Preferably, the silicone according to the invention is a non-volatile linear polydimethylsiloxane (PDMS), i.e. R₁, R₂, R₃, R₄, R₅ and R₆ are identical and denote a methyl radical.

In a first variant of the invention, the polydimethylsiloxane comprises trimethylsilyl end groups, i.e. X denotes a methyl group.

In a second variant of the invention, the polydimethylsiloxane comprises dimethylsilanol end groups, i.e. X denotes a hydroxyl group.

Examples that may be mentioned include the following polydimethylsiloxanes:
Wacker-Belsil DM 350 sold by the company Wacker
Xiameter PMX 200 Silicone Fluid 350Cs sold by the company Dow Corning
Xiameter PMX 200 Silicone Fluid 1000 Cs sold by the company Dow Corning
Wacker Belsil DM 30000 sold by the company Wacker
Wacker Belsil DM 60000 sold by the company Wacker
Mirasil DM350 sold by the company Bluestar

Mention may also be made of polydimethylsiloxanes possessing dimethylsilanol end groups known under the name of dimethiconol (CTFA), such as oils of the 48 series from Rhodia.

Among the silicones containing aryl groups are polydiarylsiloxanes, especially polydiphenylsiloxanes and polyalkylarylsiloxanes. Mention may be made, by way of example, of the products sold under the following names:
the Silbione^{®} oils of the 70 641 series from Rhodia;
the oils of the Rhodorsil^{®} 70 633 and 763 series from Rhodia;
the oil Dow Corning 556 Cosmetic Grade Fluid from Dow Corning;
the silicones of the PK series from Bayer, such as the product PK20;
certain oils of the SF series from General Electric, such as SF 1023, SF 1154, SF 1250 and SF 1265.

The total amount of non-volatile linear silicones, when they are present in the final composition, is between 0.01% and 10% and preferably between 0.02% and 5% by weight relative to the total weight of the composition.

According to one preferred embodiment of the invention, the weight ratio of the amount of non-volatile linear silicone(s) to the amount of amphoteric polymer(s) ranges from 0.5 to 30, more preferentially from 1 to 25 and more preferably from 1 to 10.

### Fatty acid diester

The composition according to the present invention may contain one or more fatty acid diesters of polyethylene glycol comprising from 2 to 50 oxyethylene units.

For the purposes of the present invention, the term "fatty compound" denotes a compound comprising a fatty chain, i.e. an alkyl or alkenyl chain comprising at least 8 carbon atoms, preferably from 8 to 30 carbon atoms and better still from 12 to 24 carbon atoms.

The fatty acid diester(s) of polyethylene glycol that may be used according to the invention advantageously have the formula (V) below: 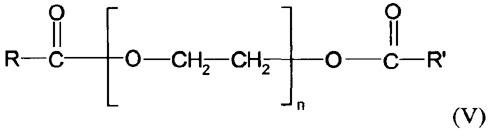
with R and R', which may be identical or different, each denoting a linear or branched alkyl or alkenyl chain comprising from 7 to 29 carbon atoms;
and n denoting an integer ranging from 2 to 50, preferably from 2 to 20 and even more preferentially from 2 to 10.

Preferably, R and R', which may be identical or different, each denote a linear or branched alkyl or alkenyl chain comprising from 11 to 23 carbon atoms, more preferably from 15 to 19 carbon atoms and better still 17 carbon atoms.

In a particularly preferred manner, the said diester is a polyethylene glycol distearate comprising from 2 to 50, preferably from 2 to 20, more preferentially from 2 to 10 and better still from 2 to 5 oxyethylene units.

The total amount of fatty acid diester(s) and of polyethylene glycol, when they are present in the final composition, is between 0.1% and 20% by weight, more preferentially between 0.5% and 10% by weight and even more preferentially between 1% and 8% by weight, relative to the total weight of the composition.

Preferably, the conditioning agent is chosen from liquid petroleum jelly, olive oil, argan oil, avocado oil, apricot kernel oil, rapeseed oil, jojoba oil, soybean oil, sunflower oil, and polyethylene glycol distearate comprising from 2 to 50, preferably from 2 to 20, more preferentially from 2 to 10 and better still from 2 to 5 oxyethylene units.

The total amount of conditioning agent(s) present in the final composition is between 0.01% and 20% by weight, more preferentially between 0.1% and 10% by weight and even more preferentially between 0.5% and 8% by weight, relative to the total weight of the composition.

According to one preferred embodiment, the composition according to the present invention also comprises one or more surfactants chosen from anionic surfactants, amphoteric or zwitterionic surfactants, nonionic surfactants and cationic surfactants.

The term "anionic surfactant" means a surfactant comprising, as ionic or ionizable groups, only anionic groups. These anionic groups are preferably chosen from CO₂H, CO₂⁻, SO₃H, SO₃⁻, OSO₃H, OSO₃⁻ , H₂PO₃, HPO₃⁻, PO₃²⁻, H₂PO₂, HPO₂⁻, PO₂²⁻ , POH and PO groups.

As examples of anionic surfactants that may be used in the composition according to the invention, mention may be made of alkyl sulfates, alkyl ether sulfates, alkylamido ether sulfates, alkylarylpolyether sulfates, monoglyceride sulfates, alkylsulfonates, alkylamidesulfonates, alkylarylsulfonates, α-olefin sulfonates, paraffin sulfonates, alkylsulfosuccinates, alkylether sulfosuccinates, alkylamide sulfosuccinates, alkylsulfoacetates, acylsarcosinates, acylglutamates, alkylsulfosuccinamates, acylisethionates and N-(C₁-C₄)alkyl N-acyltaurates, salts of alkyl monoesters of polyglycoside-polycarboxylic acids, acyllactylates, D-galactoside uronic acid salts, alkyl ether carboxylic acid salts, alkylaryl ether carboxylic acid salts, alkylamido ether carboxylic acid salts; and the corresponding non-salified forms of all these compounds; the alkyl and acyl groups of all these compounds (unless otherwise mentioned) comprising from 6 to 24 carbon atoms and the aryl group denoting a phenyl group.

These compounds may be oxyethylenated and then preferably comprise from 1 to 50 ethylene oxide units.

The salts of C₆-C₂₄ alkyl monoesters of polyglycoside-polycarboxylic acids may be selected from C₆-C₂₄ alkyl polyglycoside-citrates, C₆-C₂₄ alkyl polyglycoside-tartrates and C₆-C₂₄ alkyl polyglycoside-sulfo succinates.

When the anionic surfactant(s) are in salt form, they may be chosen from alkali metal salts such as the sodium or potassium salt and preferably the sodium salt, the ammonium salts, the amine salts and in particular the amino alcohol salts or the alkaline-earth metal salts such as the magnesium salt.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts, and in particular sodium or magnesium salts, are preferably used.

The anionic surfactants that may be present may be mild anionic surfactants, i.e. anionic surfactants without a sulfate function.

As regards the mild anionic surfactants, mention may be made in particular of the following compounds and salts thereof, and also mixtures thereof:
polyoxyalkylenated alkyl ether carboxylic acids;
polyoxyalkylenated alkylaryl ether carboxylic acids;
polyoxyalkylenated alkylamido ether carboxylic acids, in particular those comprising 2 to 50 ethylene oxide groups;
alkyl-D-galactoside uronic acids;
acylsarcosinates, acylglutamates; and
alkylpolyglycoside carboxylic esters.

Mention may be made most particularly of polyoxyalkylenated carboxylic acid alkyl ethers, for instance carboxylic acid lauryl ether (4.5 OE) sold, for example, under the name Akypo RLM 45 CA from Kao.

Preferably, the anionic surfactants are chosen from alkyl sulfates, alkyl ether sulfates, alkylamido ether sulfates, alkylsulfonates, alkylamide sulfonates, alkyl ether carboxylic acids, and N-(C₁-C₄)alkyl N-acyltaurates in which the acyl group comprises from 12 to 20 carbon atoms, in particular in the form of alkali metal or alkaline-earth metal, ammonium, amine or amino alcohol salts, and mixtures thereof, and more preferentially chosen from N-(C₁-C₄)alkyl N-acyltaurates in which the acyl group comprises from 12 to 20 carbon atoms, in the form of alkali metal or alkaline-earth metal salts.

The anionic surfactant(s) may be present in the composition according to the invention in a content ranging from 1% to 25% by weight and preferably from 3% to 20% by weight relative to the total weight of the composition.

The amphoteric or zwitterionic surfactant(s) that may be used in the present invention may especially be optionally quaternized secondary or tertiary aliphatic amine derivatives, in which the aliphatic group is a linear or branched chain containing from 8 to 22 carbon atoms, the said amine derivatives containing at least one anionic group, for instance a carboxylate, sulfonate, sulfate, phosphate or phosphonate group.

Mention may be made in particular of (C₈-C₂₀)alkylbetaines, sulfobetaines, (C₈-C₂₀ alkyl)amido(C₃-₈ alkyl)betaines and (C₈-C₂₀ alkyl)amido(C₆-C₈ alkyl)sulfobetaines.

Among the optionally quaternized secondary or tertiary aliphatic amine derivatives that can be used, as defined above, mention may also be made of the compounds of respective structures (A1) and (A2) below:

Ra-CONHCH₂CH₂- N⁺(Rb)(Rc)(CH₂COO-) (A1)

in which:
Ra represents a C₁₀-C₃₀ alkyl or alkenyl group derived from an acid Ra-COOH preferably present in hydrolysed coconut oil, a heptyl, nonyl group or undecyl group,
Rb represents a β-hydroxyethyl group, and
Rc represents a carboxymethyl group;
and

Ra'-CONHCH₂CH₂-N(B)(B') (A2)

in which:
B represents -CH₂CH₂OX',
B' represents -(CH₂)z-Y', with z = 1 or 2,
X' represents the group -CH₂-COOH, CH₂-COOZ', -CH₂CH₂-COOH, -CH₂CH₂-COOZ', or a hydrogen atom,
Y' represents -COOH, -COOZ', the group -CH₂-CHOH-SO₃H or -CH₂-CHOH-SO₃Z',
Z' represents an ion derived from an alkali metal or alkaline-earth metal, such as sodium, an ammonium ion or an ion derived from an organic amine.
Ra' represents a C₁₀-C₃₀ alkyl or alkenyl group of an acid Ra'-COOH which is preferably present in coconut oil or in hydrolysed linseed oil, or an alkyl group, especially a C₁₇ group, and its iso form, or an unsaturated C₁₇ group.

These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol^{®} C2M Concentrate.

Among the amphoteric or zwitterionic surfactants mentioned above, use is preferably made of (C₈-C₂₀ alkyl)betaines such as cocoylbetaine, and (C₈-C₂₀ alkyl)amido(C₃-C₈ alkyl)betaines such as cocamidopropylbetaine, and mixtures thereof. More preferentially, the amphoteric or zwitterionic surfactant(s) are chosen from cocoylamidopropylbetaine and cocoylbetaine.

When they are present, the amount of the amphoteric or zwitterionic surfactant(s) preferably varies within the range from 0.1% to 15% by weight, better still from 0.5% to 10% by weight and even better still from 1% to 8% by weight relative to the total weight of the composition.

Examples of nonionic surfactants that may be used in the compositions of the present invention are described, for example, in the Handbook of Surfactants by M.R. Porter, published by Blackie & Son (Glasgow and London), 1991, pp. 116-178. They are especially chosen from fatty alcohols, fatty α-diols, fatty (C₁₋₂₀)alkylphenols and fatty acids, these compounds being polyethoxylated, polypropoxylated or polyglycerolated and containing at least one fatty chain comprising, for example, from 8 to 18 carbon atoms, the number of ethylene oxide or propylene oxide groups possibly ranging especially from 2 to 50, and the number of glycerol groups possibly ranging especially from 2 to 30.

Mention may also be made of condensates of ethylene oxide and of propylene oxide with fatty alcohols; polyethoxylated fatty amides preferably having from 2 to 30 ethylene oxide units, polyglycerolated fatty amides containing on average 1 to 5, and in particular 1.5 to 4 glycerol groups, ethoxylated fatty acid esters of sorbitan containing from 2 to 30 ethylene oxide units, fatty acid esters of sucrose, fatty acid esters of polyethylene glycol, (C₆₋₂₄ alkyl)polyglycosides, N-(C₆₋₂₄ alkyl)glucamine derivatives, amine oxides such as (C₁₀₋₁₄ alkyl)amine oxides or N-(C₁₀₋₁₄ acyl)aminopropylmorpholine oxides.

When they are present, the amount of the nonionic surfactant(s) preferably ranges from 0.01% to 20% by weight and better still from 0.1% to 10% by weight relative to the total weight of the composition.

The cationic surfactant(s) that may be used in the composition according to the present invention comprise in particular salts of optionally polyoxyalkylenated primary, secondary or tertiary fatty amines, quaternary ammonium salts, and mixtures thereof.

Examples of quaternary ammonium salts that may especially be mentioned include:
- those corresponding to the general formula (VII) below: 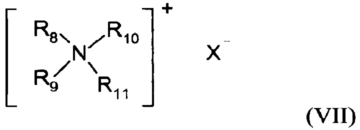 in which the groups R₈ to R₁₁, which may be identical or different, represent a linear or branched aliphatic group comprising from 1 to 30 carbon atoms or an aromatic group such as aryl or alkylaryl, at least one of the groups R₈ to R₁₁ comprising from 8 to 30 carbon atoms and preferably from 12 to 24 carbon atoms. The aliphatic groups may comprise heteroatoms such as, in particular, oxygen, nitrogen, sulfur and halogens.

The aliphatic groups are chosen, for example, from C₁-C₃₀ alkyl, C₁-C₃₀ alkoxy, polyoxy(C₂-C₆)alkylene, C₁-C₃₀ alkylamide, (C₁₂-C₂₂)alkylamido(C₂-C₆)alkyl, (C₁₂-C₂₂)alkylacetate, C₁-C₃₀ hydroxyalkyl, X⁻ is an anionic counterion chosen from halides, phosphates, acetates, lactates, (C₁-C₄)alkyl sulfates, and (C₁-C₄)alkyl-or (C₁-C₄)alkylarylsulfonates.

Preference is given, among the quaternary ammonium salts of formula (VII), on the one hand, to tetraalkylammonium chlorides, such as, for example, dialkyldimethylammonium or alkyltrimethylammonium chlorides in which the alkyl group comprises approximately from 12 to 22 carbon atoms, in particular behenyltrimethylammonium chloride, distearyldimethylammonium chloride, cetyltrimethylammonium chloride or benzyldimethylstearylammonium chloride, or also, on the other hand, to distearoylethylhydroxyethylmethylammonium methosulfate, dipalmitoylethylhydroxyethylammonium methosulfate or distearoylethylhydroxyethylammonium methosulfate, or also, finally, to palmitylamidopropyltrimethylammonium chloride or stearamidopropyldimethyl(myristyl acetate)ammonium chloride, sold under the name Ceraphyl^{®} 70 by the company Van Dyk.
- quaternary ammonium salts of imidazoline, for instance those of formula (VIII) below: 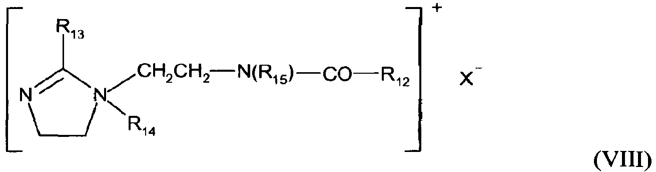 in which:
   R₁₂ represents an alkenyl or alkyl group comprising from 8 to 30 carbon atoms, for example fatty acid derivatives of tallow;
   R₁₃ represents a hydrogen atom, a C₁-C₄ alkyl group or an alkenyl or alkyl group comprising from 8 to 30 carbon atoms,
   R₁₄ represents a C₁-C₄ alkyl group,
   R₁₅ represents a hydrogen atom, a C₁-C₄ alkyl group, X⁻ is an anion chosen from the group of halides, phosphates, acetates, lactates, (C₁-C₄)alkyl sulfates and (C₁-C₄)alkyl- or (C₁-C₄)alkylarylsulfonates.
   R₁₂ and R₁₃ preferably denote a mixture of alkenyl or alkyl groups comprising from 12 to 21 carbon atoms, for example tallow fatty acid derivatives, R₁₄ denotes a methyl group, and R₁₅ denotes a hydrogen atom. Such a product is, for example, sold under the name Rewoquat^{®} W 75 by the company Rewo;
- quaternary diammonium or triammonium salts, in particular of formula (IX) below: 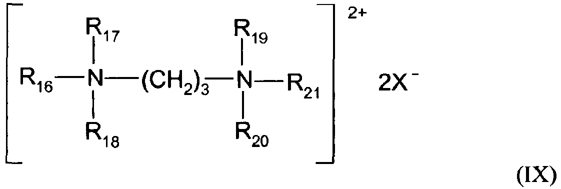
   in which R₁₆ denotes an alkyl group comprising approximately from 16 to 30 carbon atoms, which is optionally hydroxylated and/or interrupted with one or more oxygen atoms;
   R₁₇ is chosen from hydrogen, an alkyl group comprising from 1 to 4 carbon atoms or a group -(CH₂)₃-N⁺(R₁₆ₐ)(R₁₇ₐ)(R₁₈ₐ),
   R₁₆ₐ, R₁₇ₐ, R₁₈ₐ, R₁₈, R₁₉, R₂₀ and R₂₁, which may be identical or different, are chosen from hydrogen and an alkyl group comprising from 1 to 4 carbon atoms; and
   X⁻ is an anionic counterion chosen from the group of halides, acetates, phosphates, nitrates, (C₁-C₄)alkyl sulfates, (C₁-C₄)alkyl- or (C₁-C₄)alkylarylsulfonates, in particular methyl sulfate and ethyl sulfate.

Such compounds are, for example, Finquat CT-P, sold by the company Finetex (Quaternium 89), and Finquat CT, sold by the company Finetex (Quaternium 75);
- quaternary ammonium salts comprising one or more ester functions, for instance those of formula (X) below: 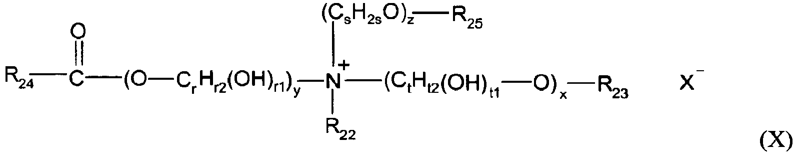 in which:
   R₂₂ is chosen from C₁-C₆ alkyl and C₁-C₆ hydroxyalkyl or dihydroxyalkyl groups,
   R₂₃ is chosen from:
      - the group 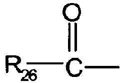
      - linear or branched, saturated or unsaturated C₁-C₂₂ hydrocarbon-based groups R₂₇,
      - a hydrogen atom,
   R₂₅ is chosen from:
      - the group 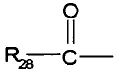
      - linear or branched, saturated or unsaturated C₁-C₆ hydrocarbon-based groups R₂₉,
      - a hydrogen atom,
   R₂₄, R₂₆ and R₂₈, which may be identical or different, are chosen from linear or branched, saturated or unsaturated C₇-C₂₁ hydrocarbon-based groups;
   r, s and t, which may be identical or different, are integers ranging from 2 to 6,
   r1 and t1, which may be identical or different, are equal to 0 or 1,
   r2 + r1 = 2 r and t1 + t2 = 2t,
   y is an integer ranging from 1 to 10,
   x and z, which may be identical or different, are integers ranging from 0 to 10,
   X⁻ is a simple or complex, organic or mineral anion,
with the proviso that the sum x + y + z is from 1 to 15, that when x is 0, then R₂₃ denotes R₂₇ and that when z is 0, then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group, and more particularly a methyl or ethyl group.

Advantageously, the sum x + y + z ranges from 1 to 10.

When R₂₃ is a hydrocarbon-based group R₂₇, it may be long and may contain from 12 to 22 carbon atoms, or may be short and may contain from 1 to 3 carbon atoms.

When R₂₅ is a hydrocarbon-based group R₂₉, it preferably contains 1 to 3 carbon atoms.

Advantageously, R₂₄, R₂₆ and R₂₈, which may be identical or different, are chosen from linear or branched, saturated or unsaturated C₁₁-C₂₁ hydrocarbon-based groups, and more particularly from linear or branched, saturated or unsaturated C₁₁-C₂₁ alkyl and alkenyl groups.

x and z, which may be identical or different, are preferably 0 or 1.

y is advantageously equal to 1.

Preferably, r, s and t, which may be identical or different, equal to 2 or 3, and even more particularly are equal to 2.

The anion X⁻ is preferably a halide, preferably chloride, bromide or iodide, a (C₁-C₄)alkyl sulfate or a (C₁-C₄)alkyl- or (C₁-C₄)alkylaryl-sulfonate. However, it is possible to use methanesulfonate, phosphate, nitrate, tosylate, an anion derived from an organic acid, such as acetate or lactate, or any other anion that is compatible with the ammonium containing an ester function.

The anion X⁻ is even more particularly chloride, methyl sulfate or ethyl sulfate.

Use is made more particularly, in the composition according to the invention, of the ammonium salts of formula (X) in which:
- R₂₂ denotes a methyl or ethyl group,
- x and y are equal to 1,
- z is equal to 0 or 1,
- r, s and t are equal to 2,
- R₂₃ is chosen from:
- the group 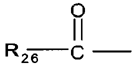
- methyl, ethyl or C₁₄-C₂₂ hydrocarbon-based groups;
- a hydrogen atom,
- R₂₅ is chosen from:
- the group 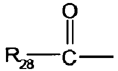
- a hydrogen atom,
- R₂₄, R₂₆ and R₂₈, which may be identical or different, are chosen from linear or branched, saturated or unsaturated C₁₃-C₁₇ hydrocarbon-based groups, and more particularly from linear or branched, saturated or unsaturated C₁₃-C₁₇ alkyl and alkenyl groups.

The hydrocarbon-based groups are advantageously linear.

Among the compounds of formula (X), examples that may be mentioned include salts, especially the chloride or methyl sulfate, of diacylo xyethyldimethylammonium, diacyloxyethylhydroxyethylmethylammonium, monoacyloxyethyldihydroxyethylmethylammonium, triacyloxyethylmethylammonium or monoacyloxyethylhydroxyethyldimethylammonium, and mixtures thereof. The acyl groups preferably contain 14 to 18 carbon atoms and are obtained more particularly from a plant oil such as palm oil or sunflower oil. When the compound contains several acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with fatty acids or with fatty acid mixtures of plant or animal origin, or by transesterification of the methyl esters thereof. This esterification is followed by a quaternization by means of an alkylating agent such as an alkyl halide, preferably methyl or ethyl halide, a dialkyl sulfate, preferably dimethyl or diethyl sulfate, methyl methanesulfonate, methyl para-toluenesulfonate, glycol chlorohydrin or glycerol chlorohydrin.

Such compounds are, for example, sold under the names Dehyquart^{®} by the company Henkel, Stepanquat^{®} by the company Stepan, Noxamium^{®} by the company Ceca or Rewoquat^{®} WE 18 by the company Rewo-Witco.

The composition according to the invention may contain, for example, a mixture of quaternary ammonium monoester, diester and triester salts with a weight majority of diester salts.

Use may also be made of the ammonium salts containing at least one ester function that are described in patents US-A-4 874 554 and US-A-4 137 180.

Use may also be made of behenoylhydroxypropyltrimethylammonium chloride sold by the company KAO under the name Quartamin BTC 131.

Preferably, the ammonium salts containing at least one ester function contain two ester functions.

Among the cationic surfactants present in the composition according to the invention, it is more particularly preferred to choose cetyltrimethylammonium, behenyltrimethylammonium and dipalmitoylethylhydroxyethylmethylammonium salts, and mixtures thereof, and more particularly behenyltrimethylammonium chloride, cetyltrimethylammonium chloride, and dipalmitoylethylhydroxyethylammonium methosulfate, and mixtures thereof.

When the composition contains one or more cationic surfactants, their content preferably ranges from 0.05% to 10% by weight, more preferentially from 0.1% to 5% by weight and better still from 0.5% to 5% by weight relative to the total weight of the composition.

According to a particularly preferred embodiment, the composition according to the invention comprises one or more anionic surfactants and one or more amphoteric or zwitterionic surfactants.

Preferably, the total amount of surfactants in the composition according to the invention ranges from 3% to 50% by weight, more preferentially from 5% to 30% by weight and better still from 8% to 25% by weight relative to the total weight of the composition.

Preferably, the composition according to the invention is aqueous and comprises at least 30% by weight and preferably at least 50% by weight of water relative to the total weight of the composition.

When the composition comprises an oil according to the invention and water, the composition according to the invention is preferentially in the form of an oil-in-water or water-in-oil emulsion.

The composition according to the invention may also contain one or more organic solvents that are liquid at room temperature (25°C) and at atmospheric pressure (760 mmHg, i.e. 1.013 × 10⁵ Pa). Preferably, the liquid organic solvent(s) are chosen from C₁-C₄ lower alcohols, such as ethanol, isopropanol, tert-butanol or n-butanol, polyols such as propylene glycol, hexylene glycol and glycerol, and polyol ethers, and mixtures thereof.

When the composition of the invention is aqueous, its pH is generally between 2 and 9 and in particular between 3 and 8. Preferably, the pH is less than 7. Even more preferentially, it ranges from 3 to 6.

It may be adjusted to the desired value by means of acidifying or basifying agents usually used in cosmetics for this type of application, or alternatively using standard buffer systems.

Among the acidifying agents, examples that may be mentioned include the organic acids already mentioned previously, or mineral acids.

The term "mineral acid" means any acid derived from a mineral compound. Among the mineral acids, mention may be made of hydrochloric acid, orthophosphoric acid, sulfuric acid, sulfonic acids and nitric acid.

Use may be made especially of mineral or organic acids such as hydrochloric acid, orthophosphoric acid, sulfuric acid, carboxylic acids, for instance acetic acid, tartaric acid, citric acid, lactic acid or malic acid, amino acids and sulfonic acids.

Among the basifying agents, examples that may be mentioned include aqueous ammonia, alkali metal carbonates, alkanolamines, such as mono-, di- and triethanolamines and derivatives thereof, amino acids, sodium hydroxide, potassium hydroxide and the compounds of the following formula: 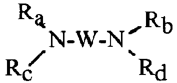 in which W is a propylene residue optionally substituted with a hydroxyl group or a C₁-C₄ alkyl group; Rₐ, R_{b}, R_{c} and R_{d}, which may be identical or different, represent a hydrogen atom or a C₁-C₄ alkyl or C₁-C₄ hydroxyalkyl group.

Preferably, the pH modifiers may be chosen from alkaline agents, such as aqueous ammonia, monoethanolamine, diethanolamine, triethanolamine, 1,3-propanediamine, 2-amino-2-methyl-1-propanol or an alkaline hydroxide, or else acidifying agents, such as phosphoric acid, hydrochloric acid or citric acid.

The compositions according to the invention may also contain one or more additives chosen from ceramides, volatile or non-volatile silicones, which may or may not be organomodified with organic groups such as quaternized or non-quaternized amino groups or thiol groups, vitamins and pro-vitamins including panthenol, water-soluble and liposoluble, silicone or non-silicone sunscreens, nacreous agents, such as distearyl ether and β-cyclodextrin, and opacifiers, sequestrants, oily or waxy fatty esters other than the plant oils, fatty alcohols, thickeners, solubilizers, antioxidants, antidandruff agents, anti-seborrhoeic agents, hair-loss counteractants and/or hair-regrowth promoters, penetrants, fragrances, peptizers and preserving agents, or any other additive conventionally used in the cosmetics field.

The thickener(s) may be chosen especially from cellulose-based thickeners, for example hydroxyethylcellulose, hydroxypropylcellulose and carboxymethylcellulose, guar gum and derivatives thereof, for example the hydroxypropyl guar sold by the company Rhodia under the reference Jaguar HP 105, gums of microbial origin, such as xanthan gum and scleroglucan gum, synthetic thickeners such as crosslinked acrylic acid or acrylamidopropanesulfonic acid homopolymers, for example Carbomer, nonionic, anionic, cationic or amphoteric associative polymers, for instance the polymers sold under the names Pemulen TR1 or TR2 by the company Goodrich, Salcare SC90 by the company Ciba, Aculyn 22, 28, 33, 44 or 46 by the company Rohm & Haas, and Elfacos T210 and T212 by the company Akzo.

These additives may be present in the composition according to the invention in an amount ranging from 0 to 20% by weight relative to the total weight of the composition.

A person skilled in the art will take care to select these optional additives and amounts thereof so that they do not harm the properties of the compositions of the present invention.

The cosmetic compositions of the invention may be transparent or translucent, i.e. these compositions allow a transmittance at 600 nm of greater than 85%, better still greater than 90% and even better still greater than 94%.

The compositions according to the invention may, in a nonlimiting manner, be in the form of shampoos, care products to be applied, where appropriate, before and/or after shampooing or dyeing or permanent-waving, or hair dyeing, bleaching, permanent-waving, relaxing or styling products.

According to a first preferred embodiment, the composition according to the invention is in the form of a shampoo.

In this case, it advantageously contains:
- one or more amphoteric polymers comprising a repetition of:
   (i) one or more units obtained from a monomer of (meth)acrylamide type,
   (ii) one or more units obtained from a monomer of (meth)acrylamidoalkyltrialkylammonium type, and
   (iii) one or more units obtained from an acidic monomer of (meth)acrylic acid type,
   the said amphoteric polymer(s) being present in the composition in an amount of between 0.01% and 10% by weight relative to the total weight of the composition,
- one or more conditioning agents chosen from plant oils, mineral oils, fatty acid diesters of polyethylene glycol comprising from 2 to 50 oxyethylene units, and a mixture thereof,
- one or more anionic surfactants in a content ranging from 3% to 20% by weight and preferably from 4% to 15% by weight relative to the total weight of the composition,
- optionally, one or more amphoteric or zwitterionic surfactants, in a content ranging from 0.1% to 15% by weight relative to the total weight of the composition, and
- optionally, one or more nonionic surfactants, in a content ranging from 0.01% to 20% by weight relative to the total weight of the composition.

In one variant of this embodiment, the composition comprises at least one N-acyl-N-(C₁-C₄) alkyltaurate, in which the acyl group comprises from 12 to 20 carbon atoms, in the form of an alkali metal or alkaline-earth metal salt.

In a second preferred embodiment, the composition according to the invention is in the form of a haircare product.

In this case, it advantageously contains:
- one or more amphoteric polymers comprising a repetition of:
   (i) one or more units obtained from a monomer of (meth)acrylamide type,
   (ii) one or more units obtained from a monomer of (meth)acrylamidoalkyltrialkylammonium type, and
   (iii) one or more units obtained from an acidic monomer of (meth)acrylic acid type,
   the said amphoteric polymer(s) being present in the composition in an amount of between 0.01% and 10% by weight relative to the total weight of the composition,
- one or more conditioning agents chosen from plant oils, mineral oils, fatty acid diesters of polyethylene glycol comprising from 2 to 50 oxyethylene units, and a mixture thereof, and
- one or more cationic surfactants in a content ranging from 0.05% to 10% by weight, relative to the total weight of the composition.

The present invention also relates to a cosmetic hair treatment process, which consists in applying to the hair an effective amount of a composition as described above.

This application may or may not be followed by a rinsing operation.

When the application of the composition is followed by rinsing, the leave-on time of the composition on the keratin materials ranges from a few seconds to 60 minutes, better still from 5 seconds to 30 minutes, even better still from 10 seconds to 10 minutes.

Whether in rinse-out mode or leave-in mode, the application of the composition may take place in the presence or absence of heat. The heating device may be a hairdryer, a hood dryer, a curling iron or a flat iron. The heating temperature may be between 40°C and 220°C.

Preferably, the compositions according to the invention are used as shampoos for washing and conditioning the hair, or as haircare products.

Even more preferentially, the compositions according to the invention are used as shampoos for washing and conditioning the hair.

The compositions according to the invention are used for facilitating the styling and/or for improving the suppleness, smoothness and/or disentangling of the hair.

The examples that follow serve to illustrate the invention without, however, being limiting in nature.

### EXAMPLES

In the following examples, all the amounts are shown as percentage by weight of active material relative to the total weight of the composition.

### Example 1: shampoos for sparingly sensitized hair

The shampoo composition A below according to the invention was prepared from the compounds indicated in the table below.

| Composition A (% AM) | |
|---|---|
| Monoisopropanolamine lauryl ether sulfate (2 OE) ⁽¹⁾ | 6.45 |
| Propylene glycol | 2 |
| Acrylic acid/methylacrylamidopropyltrimethylammonium chloride (MAPTAC)/acrylamide terpolymer (Polyquaternium-53) ⁽²⁾ | 1 |
| Castor oil PEG-18 dioleate | 2 |
| Avocado oil | 1 |
| Glycerol | 2 |
| Sodium chloride | 2.5 |
| Sodium benzoate | 0.5 |
| Fragrance | qs |
| Lauryl ether carboxylic acid (4.5 OE) ⁽³⁾ | 0.9 |
| Cocoylamidopropylbetaine | 3.04 |
| PEG-55 propylene glycol oleate ⁽⁴⁾ | 0.8 |
| Sodium lauryl ether sulfate (2.2 OE) | 9.8 |
| pH agent qs | 5.3 |
| Water qs | 100% |
| ⁽¹⁾ sold under the trade name Marlinat 242/90 M by the company Sasol | |
| ⁽²⁾ sold under the trade name Merquat 2003PR by the company Nalco | |
| ⁽³⁾ sold under the trade name Akypo RLM 45 by the company Kao | |
| ⁽⁴⁾ sold under the trade name Antil 141 Liquid by the company Evonik Goldschmidt | |

Composition A was tested by hairstyling experts. These experts demonstrated the provision of a good level of smoothness and of disentangling, while at the same time maintaining lightness and ease of styling without any lank effect.

### Example 2: shampoos for sparingly sensitized hair

Compositions B and C below according to the invention were prepared from the compounds indicated in the table below.

| Compositions (% AM) | B | C |
|---|---|---|
| Monoisopropanolamine lauryl ether sulfate (2 OE) ⁽¹⁾ | 6.45 | 6.45 |
| Acrylic acid/methylacrylamidopropyltrimethylam monium chloride (MAPTAC)/acrylamide terpolymer ⁽²⁾ | 0.2 | 0.2 |
| Castor oil PEG-18 dioleate | 2 | 2 |
| Apricot kernel oil | 1 | - |
| Camelina oil | - | 1 |
| Glycerol | 2 | 2 |
| Sodium chloride | 2.5 | 2.5 |
| Sodium benzoate | 0.5 | 0.5 |
| Fragrance | qs | qs |
| Lauryl ether carboxylic acid (4.5 OE) ⁽³⁾ | 0.9 | 0.9 |
| Cocoylamidopropylbetaine | 3.04 | 3.04 |
| PEG-55 propylene glycol oleate ⁽⁴⁾ | 0.8 | 0.8 |
| Sodium lauryl ether sulfate (2.2 OE) | 9.8 | 9.8 |
| pH agent qs | 5.3 | 5.3 |
| Water qs | 100% | 100% |
| ⁽¹⁾ sold under the trade name Marlinat 242/90 M by the company Sasol | | |
| ⁽²⁾ sold under the trade name Merquat 2003PR by the company Nalco | | |
| ⁽³⁾ sold under the trade name Akypo RLM 45 by the company Kao | | |
| ⁽⁴⁾ sold under the trade name Antil 141 Liquid by the company Evonik Goldschmidt | | |

The head tests performed with compositions B and C lead to good cosmeticity, in terms of smoothness and disentangling, without any sensation of a lank effect.

### Example 5: shampoo

A shampoo composition F according to the invention was prepared from the compounds indicated in the table below.

| Composition F (g% AM) | |
|---|---|
| Sodium lauryl sulfate | 4 |
| Sodium lauryl ether sulfate (2.2 OE) | 11.9 |
| Acrylic acid/MAPTAC/acrylamide terpolymer (10/40/50)⁽¹⁾ | 0.5 |
| Zinc pyrithione⁽²⁾ | 1 |
| Carboxyvinyl polymer⁽³⁾ | 0.3 |
| Coconut acid monoethanolamide | 1.8 |
| Polyethylene glycol distearate 3 OE | 1.5 |
| Dimethicone | 0.75 |
| Glycerol | 2 |
| Sodium benzoate | 0.5 |
| Sodium chloride | 1.1 |
| Salicylic acid | 0.2 |
| Fragrance | qs |
| pH agent | qs pH = 5.3 |
| Water | qs 100% |
| ⁽¹⁾ sold under the trade name Merquat 2003PR by the company Nalco | |
| ⁽²⁾ sold under the trade name Zinc omadine pyrithione 48% DSP COSM NE by the company Arch Chemical | |
| ⁽³⁾ sold under the trade name Carbopol 980 Polymer by the company Lubrizol | |

The shampoo composition F obtained is stable over time, both at room temperature and at 45°C.

This composition is used as a shampoo, by applying it to wet hair, and then, after working it into a lather and leaving it to stand on the hair for a few minutes, the composition is removed from the head of hair by rinsing with water.

This composition was tested by experts, who found that it leads to good cosmetic properties, and especially to good conditioning properties in terms of smoothness and disentangling, while at the same time maintaining good lightness of the hair, without a lank effect. The hair is also easier to style.

In addition, the composition has a particularly aesthetic nacreous appearance (almost metallized shiny pearlescence).

## Claims

1. Cosmetic composition comprising:
a)- one or more amphoteric polymers comprising a repetition of:
(i) one or more units obtained from a monomer of (meth)acrylamide type,
(ii) one or more units obtained from a monomer of (meth)acrylamidoalkyltrialkylammonium type, and
(iii) one or more units obtained from an acidic monomer of (meth)acrylic acid type,
the said amphoteric polymer(s) being present in the composition in an amount of between 0.01% and 10% by weight relative to the total weight of the composition, and
b)- one or more conditioning agents chosen from plant oils, mineral oils, fatty acid diesters of polyethylene glycol comprising from 2 to 50 oxyethylene units, and a mixture thereof,
the composition may further comprise additional conditioning agents chosen from non-volatile linear silicones of polydialkylsiloxane structure, non-volatile linear silicones of polydiarylsiloxane structure, and non-volatile linear silicones of polyalkylarylsiloxane structure;
the total amount of said conditioning agent(s) present in the composition being between 0.01% and 20% by weight, relative to the total weight of the composition.

2. Composition according to Claim 1, **characterized in that** the amphoteric polymer comprises from 30 mol% to 70 mol% of units derived from a monomer of (meth)acrylamide type, from 10 mol% to 60 mol% of units derived from a monomer of (meth)acrylamidoalkyltrialkylammonium type and from 1 mol% to 20 mol% of units derived from an acidic monomer of (meth)acrylic acid type.

3. Composition according to Claim 1 or 2, **characterized in that** the amphoteric polymer comprises units derived from the following monomers: (i) acrylamide, (ii) acrylamidopropyltrimethylammonium chloride, and (iii) acrylic acid.

4. Composition according to any one of the preceding claims, **characterized in that** the amphoteric polymer is present in the composition in an amount of between 0.02% and 10% by weight and preferably between 0.05% and 5% by weight relative to the total weight of the composition.

5. Composition according to any one of the preceding claims, **characterized in that** the conditioning agent is chosen from the following plant oils:
- sweet almond oil,
- apricot kernel oil,
- argan oil,
- babassu oil,
- avocado oil,
- groundnut oil,
- candlenut oil,
- camellia oil,
- camelina oil,
- safflower oil,
- beauty-leaf oil,
- rapeseed oil,
- coconut oil,
- coriander oil,
- marrow oil,
- wheatgerm oil,
- jojoba oil,
- linseed oil,
- macadamia oil,
- corn germ oil,
- hazelnut oil,
- walnut oil,
- vernonia oil,
- olive oil,
- evening primrose oil,
- palm oil,
- passion flower oil,
- grapeseed oil,
- pracaxi oil,
- rose oil,
- castor oil,
- rye oil,
- sesame oil,
- rice bran oil,
- soybean oil,
- tamanu oil, and
- sunflower oil.

6. Composition according to any one of Claims 1 to 4, **characterized in that** the conditioning agent is chosen from the following mineral oils:
- mixtures of hydrocarbon-based oils derived from petroleum,
- volatile or non-volatile liquid paraffin,
- liquid petroleum jelly,
- polyolefins and in particular polydecenes,
- isoparaffins such as isohexadecane, isododecane and hydrogenated polyisobutylenes.

7. Composition according to any one of Claims 1 to 4, **characterized in that** the conditioning agent is chosen from the fatty acid dimer esters of polyethylene glycol of formula (V) below: 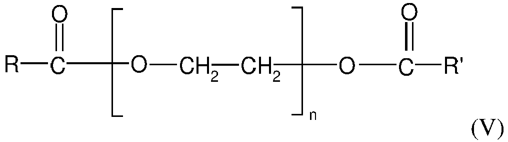
with R and R', which may be identical or different, each denoting a linear or branched alkyl or alkenyl chain comprising from 7 to 29 carbon atoms, preferably from 11 to 23 carbon atoms, more preferably from 15 to 19 carbon atoms and better still 17 carbon atoms;
and n denoting an integer ranging from 2 to 50, preferably from 2 to 20 and even more preferentially from 2 to 10.

8. Composition according to any one of the preceding claims, **characterized in that** the conditioning agent is chosen from liquid petroleum jelly, olive oil, argan oil, avocado oil, apricot kernel oil, rapeseed oil, jojoba oil, soybean oil, sunflower oil, and polyethylene glycol distearate comprising from 2 to 50, preferably from 2 to 20, more preferentially from 2 to 10 and better still from 2 to 5 oxyethylene units.

9. Composition according to any one of the preceding claims, **characterized in that** the total amount of conditioning agent(s) present in the final composition is between 0.1% and 10% by weight and even more preferentially between 0.5% and 8% by weight, relative to the total weight of the composition.

10. Composition according to any one of the preceding claims, **characterized in that** it also comprises one or more surfactants chosen from anionic surfactants, amphoteric or zwitterionic surfactants, nonionic surfactants and cationic surfactants.

11. Composition according to the preceding claim, **characterized in that** it comprises one or more anionic surfactants in a content ranging from 1% to 25% by weight and preferably from 3% to 20% by weight relative to the total weight of the composition, preferably chosen from alkyl sulfates, alkyl ether sulfates, alkylamido ether sulfates, alkylsulfonates, alkylamidesulfonates, alkyl ether carboxylic acids, N-(C₁-C₄)alkyl N-acyltaurates in which the acyl group comprises from 12 to 20 carbon atoms, in particular in the form of alkali metal or alkaline-earth metal, ammonium, amine or amino alcohol salts, and mixtures thereof, and more preferentially chosen from N-(C₁-C₄)alkyl N-acyltaurates in which the acyl group comprises from 12 to 20 carbon atoms, in the form of alkali metal or alkaline-earth metal salts.

12. Composition according to Claim 10 or 11, **characterized in that** it comprises one or more amphoteric or zwitterionic surfactants in a content ranging from 0.1% to 15% by weight, better still from 0.5% to 10% by weight and even better still from 1% to 8% by weight, relative to the total weight of the composition, preferably chosen from (C₈₋₂₀ alkyl)betaines and (C₈₋₂₀ alkyl)amido(C₃₋₈ alkyl)betaines, and mixtures thereof.

13. Cosmetic process for treating keratin materials, especially human keratin fibres such as the hair, **characterized in that** it consists in applying to the keratin materials a composition according to any one of Claims 1 to 12 and then in optionally rinsing it out after an optional leave-on time, in the presence or absence of heat.

14. Use of a composition according to any one of Claims 1 to 12, as a shampoo for cleansing and conditioning keratin fibres.

15. Use according to Claim 14, for facilitating the styling and/or for improving the suppleness, smoothness and/or disentangling of the hair.

## Patentansprüche

1. Kosmetische Zusammensetzung, umfassend:
a) - ein oder mehrere amphotere Polymere, umfassend eine Wiederholung von:
(i) einer oder mehreren Einheiten, die aus einem Monomer vom (Meth)acrylamidtyp erhalten werden,
(ii) einer oder mehreren Einheiten, die aus einem Monomer vom (Meth)acrylamidoalkyl-trialkylammoniumtyp erhalten werden, und
(iii) einer oder mehreren Einheiten, die aus einem sauren Monomer vom (Meth)acrylsäuretyp erhalten werden,
wobei das amphotere Polymer bzw. die amphoteren Polymere in der Zusammensetzung in einer Menge zwischen 0,01 Gew.% und 10 Gew.% vorhanden ist bzw. sind, bezogen auf das Gesamtgewicht der Zusammensetzung, und
b) - ein oder mehrere Konditionierungsmittel ausgewählt aus Pflanzenölen, Mineralölen, Fettsäurediestern von Polyethylenglykol, die 2 bis 50 Oxyethyleneinheiten umfassen, und einer Mischung davon,
wobei die Zusammensetzung außerdem zusätzlich Konditionierungsmittel umfassen kann, die nichtflüchtigen linearen Silikonen mit Polydialkylsiloxanstruktur, nichtflüchtigen linearen Silikonen mit Polydiarylsiloxanstruktur, und nichtflüchtigen linearen Silikonen mit Polyalkylarylsiloxanstruktur ausgewählt sind,
wobei die Gesamtmenge des/der Konditionierungsmittel(s), das bzw. die in der Zusammensetzung vorhanden ist bzw. sind, 0,01 Gew.% bis 20 Gew.% beträgt, bezogen auf das Gesamtgewicht der Zusammensetzung.

2. Zusammensetzung nach Anspruch 1, **dadurch gekennzeichnet, dass** das amphotere Polymer 30 Mol% bis 70 Mol% Einheiten, die von einem Monomer vom (Meth)acrylamidtyp abgeleitet sind, 10 Mol% bis 60 Mol% Einheiten, die von einem Monomer vom (Meth)-acrylamidoalkyltrialkylammoniumtyp abgeleitet sind, und 1 Mol% bis 20 Mol% Einheiten umfasst, die von einem sauren Monomer vom (Meth)acrylsäuretyp abgeleitet sind.

3. Zusammensetzung nach Anspruch 1 oder 2, **dadurch gekennzeichnet, dass** das amphotere Polymer Einheiten umfasst, die von den folgenden Monomeren abgeleitet sind: (i) Acrylamid, (ii) Acrylamidopropyltrimethylammoniumchlorid und (iii) Acrylsäure.

4. Zusammensetzung nach einem der vorhergehenden Ansprüche, **dadurch gekennzeichnet, dass** das amphotere Polymer in der Zusammensetzung in einer Menge zwischen 0,02 Gew.% und 10 Gew.% und vorzugsweise zwischen 0,05 Gew.% und 5 Gew.% vorhanden ist, bezogen auf das Gesamtgewicht der Zusammensetzung.

5. Zusammensetzung nach einem der vorhergehenden Ansprüche, **dadurch gekennzeichnet, dass** das Konditionierungsmittel aus den folgenden Pflanzenölen ausgewählt ist:
- süßem Mandelöl,
- Aprikosenkernöl,
- Arganöl,
- Babassuöl
- Avocadoöl,
- Erdnussöl,
- Kerzennussöl
- Kamelienöl,
- Leindotteröl,
- Distelöl
- alexandrinischem Lorbeeröl,
- Rapsöl,
- Kokosnussöl
- Korianderöl,
- Marköl,
- Weizenkeimöl,
- Jojobaöl,
- Leinsamenöl,
- Macadamiaöl,
- Maiskeimöl,
- Haselnussöl,
- Walnussöl,
- Vernoniaöl,
- Olivenöl,
- Nachtkerzenöl,
- Palmöl,
- Passionsblumenöl,
- Traubenkernöl,
- Pracaxiöl,
- Rosenöl,
- Castoröl
- Roggenöl,
- Sesamöl,
- Reiskleieöl,
- Sojaöl,
- Tamanuöl und
- Sonnenblumenöl.

6. Zusammensetzung nach einem der Ansprüche 1 bis 4, **dadurch gekennzeichnet, dass** das Konditionierungsmittel aus den folgenden Mineralölen ausgewählt ist:
- Mischungen von Ölen auf Kohlenwasserstoffbasis, die abgeleitet sind von Erdöl,
- flüchtigem oder nichtflüchtigem Paraffin,
- flüssigem Petrolatum,
- Polyolefinen und insbesondere Polydecenen,
- Isoparaffinen, wie Isohexadecan, Isododecan und hydrierten Polyisobutylenen.

7. Zusammensetzung nach einem der Ansprüche 1 bis 4, **dadurch gekennzeichnet, dass** das Konditionierungsmittel aus den Fettsäuredimerestern von Polyethylenglykol mit der folgenden Formel (V) ausgewählt ist: 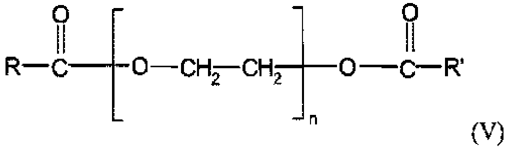
wobei R und R', die gleich oder unterschiedlich sein können, jeweils eine lineare oder verzweigte Alkyl- oder Alkenylkette bezeichnen, die 7 bis 29 Kohlenstoffatome, vorzugsweise 11 bis 23 Kohlenstoffatome, bevorzugter 15 bis 19 Kohlenstoffatome und noch besser 17 Kohlenstoffatome umfasst;
und n eine ganze Zahl im Bereich von 2 bis 50, vorzugsweise 2 bis 20 und bevorzugter 2 bis 10 bezeichnet.

8. Zusammensetzung nach einem der vorhergehenden Ansprüche, **dadurch gekennzeichnet, dass** das Konditionierungsmittel ausgewählt ist aus flüssigem Petrolatum, Olivenöl, Arganöl, Avocadoöl, Aprikosenkernöl, Rapsöl, Jojobaöl, Sojaöl, Sonnenblumenöl und Polyethylenglykoldistearat, umfassend 2 bis 50, vorzugsweise 2 bis 20, noch bevorzugter 2 bis 10 und besser noch 2 bis 5 Oxyethyleneinheiten.

9. Zusammensetzung nach einem der vorhergehenden Ansprüche, **dadurch gekennzeichnet, dass** die Gesamtmenge an Konditionierungsmittel(n), das bzw. die in der Endzusammensetzung enthalten ist bzw. sind, zwischen 0,1 Gew.% und 10 Gew.% und noch bevorzugter zwischen 0,5 Gew.% und 8 Gew.% liegt, bezogen auf das Gesamtgewicht der Zusammensetzung.

10. Zusammensetzung nach einem der vorhergehenden Ansprüche, **dadurch gekennzeichnet, dass** sie auch ein oder mehrere Tenside ausgewählt aus anionischen Tensiden, amphoteren oder zwitterionischen Tensiden, nichtionischen Tensiden und kationischen Tensiden umfasst.

11. Zusammensetzung nach dem vorhergehenden Anspruch, **dadurch gekennzeichnet, dass** sie ein oder mehrere anionische Tenside in einem Gehalt im Bereich von 1 Gew.% bis 25 Gew.% und vorzugsweise 3 Gew.% bis 20 Gew.% umfasst, bezogen auf das Gesamtgewicht der Zusammensetzung, vorzugsweise ausgewählt aus Alkylsulfaten, Alkylethersulfaten, Alkylamidoethersulfaten, Alkylsulfonaten, Alkylamidsulfonaten, Alkylethercarbonsäuren, N-(C₁-C₄)Alkyl-N-acyltauraten, bei denen die Acylgruppe 12 bis 20 Kohlenstoffatome umfasst, insbesondere in Form von Alkalimetall- oder Erdalkalimetall-, Ammonium-, Amin- oder Aminoalkoholsalzen und Mischungen davon und bevorzugter ausgewählt aus N-(C₁-C₄)Alkyl-N-acyltauraten, in denen die Acylgruppe 12 bis 20 Kohlenstoffatome umfasst, in Form von Alkalimetall- oder Erdalkalimetallsalzen.

12. Zusammensetzung nach Anspruch 10 oder 11, **dadurch gekennzeichnet, dass** sie ein oder mehrere amphotere oder zwitterionische Tenside in einem Gehalt im Bereich von 0,1 Gew.% bis 15 Gew.%, besser noch 0,5 Gew.% bis 10 Gew.% und noch besser von 1 Gew.% bis 8 Gew.% umfasst, bezogen auf das Gesamtgewicht der Zusammensetzung, vorzugsweise ausgewählt aus (C₈-₂₀-Alkyl) betainen und (C₈-₂₀-Alkyl)amido(C₃₋₈-alkyl)betainen und Mischungen davon.

13. Kosmetisches Verfahren zum Behandeln von Keratinmaterialien, insbesondere menschlichen Keratinfasern, wie dem Haar, **dadurch gekennzeichnet, dass** es darin besteht, auf die Keratinmaterialien eine Zusammensetzung gemäß einem der Ansprüche 1 bis 13 aufzutragen und sie dann gegebenenfalls in Gegenwart oder Abwesenheit von Wärme nach einer optionalen Zeit, in der sie darauf verbleiben, auszuspülen.

14. Verwendung einer Zusammensetzung nach einem der Ansprüche 1 bis 12 als Shampoo zur Reinigung und Konditionierung von Keratinfasern.

15. Verwendung nach Anspruch 14 zur Erleichterung des Frisierens und/oder zur Verbesserung von Geschmeidigkeit, Glätte und/oder Entwirren des Haares.

## Revendications

1. Composition cosmétique comprenant :
a) un ou plusieurs polymère(s) amphotère(s) comprenant la répétition de :
(i) un ou plusieurs motif(s) issu(s) d'un monomère de type (méth)acrylamide,
(ii) un ou plusieurs motif(s) issu(s) d'un monomère de type (méth)acrylamidoalkyl-trialkyl-ammonium, et
(iii) un ou plusieurs motif(s) issu(s) d'un monomère acide de type acide (méth)acrylique,
le ou lesdits polymère(s) amphotère(s) étant présent(s) dans la composition en une proportion comprise entre 0,01 et 10% en poids, par rapport au poids total de la composition, et
b) un ou plusieurs agent(s) de conditionnement choisi(s) parmi les huiles végétales, les huiles minérales, les diesters d'acide gras et de polyéthylèneglycol comportant de 2 à 50 motifs oxy-éthylène, et leurs mélanges,
la composition peut en outre comprendre des agents de conditionnement additionnels choisis parmi les silicones linéaires non-volatiles à structure de type poly(dialkyl-siloxane), les silicones linéaires non-volatiles à structure de type poly(diaryl-siloxane), les silicones linéaires non-volatiles à structure de type poly(alkyl-aryl-siloxane),
étant entendu que la proportion totale du ou desdits agent(s) de conditionnement présent(s) dans la composition est comprise entre 0,01 et 20 % en poids, par rapport au poids total de la composition.

2. Composition selon la revendication 1, **caractérisée en ce que** le polymère amphotère comprend de 30 à 70 % en moles de motifs dérivés d'un monomère de type (méth)acrylamide, de 10 à 60 % en moles de motifs dérivés d'un monomère de type (méth)acrylamidoalkyl-trialkyl-ammonium, et de 1 à 20 % en moles de motifs dérivés d'un monomère acide de type acide (méth)acrylique.

3. Composition selon la revendication 1 ou 2, **caractérisée en ce que** le polymère amphotère comprend des motifs dérivés des monomères suivants : (i) acrylamide, (ii) chlorure d'acrylamidopropyl-triméthyl-ammonium, et (iii) acide acrylique.

4. Composition selon l'une quelconque des revendications précédentes, **caractérisée en ce que** le polymère amphotère est présent dans la composition en une proportion comprise entre 0,02 et 10 % en poids, et de préférence entre 0,05 et 5 % en poids, par rapport au poids total de la composition.

5. Composition selon l'une quelconque des revendications précédentes, **caractérisée en ce que** l'agent de conditionnement est choisi parmi les huiles végétales suivantes :
- l'huile d'amande douce,
- l'huile d'amande d'abricot,
- l'huile d'argan,
- l'huile de babassu,
- l'huile d'avocat,
- l'huile d'arachide,
- l'huile de bancoulier,
- l'huile de camélia,
- l'huile de camélina,
- l'huile de carthame,
- l'huile de calophyllum,
- l'huile de colza,
- l'huile de coprah,
- l'huile de coriandre,
- l'huile de courge,
- l'huile de germes de blé,
- l'huile de jojoba,
- l'huile de lin,
- l'huile de macadamia,
- l'huile de germes de maïs,
- l'huile de noisette,
- l'huile de noix,
- l'huile de vernonia,
- l'huile d'olive,
- l'huile d'onagre,
- l'huile de palme,
- l'huile de passiflore,
- l'huile de pépins de raisin,
- l'huile de pracaxi,
- l'huile de rosier,
- l'huile de ricin,
- l'huile de seigle,
- l'huile de sésame,
- l'huile de son de riz,
- l'huile de soja,
- l'huile de tamanu,
- et l'huile de tournesol.

6. Composition selon l'une quelconque des revendications 1 à 4, **caractérisée en ce que** l'agent de conditionnement est choisi parmi les huiles minérales suivantes :
- les mélanges d'huiles hydrocarbonées dérivées du pétrole,
- l'huile de paraffine, volatile ou non volatile,
- l'huile de vaseline,
- les polyoléfines et en particulier les polydécènes,
- et les isoparaffines telles que l'isohexadécane, l'isododécane et les polyisobutylènes hydrogénés.

7. Composition selon l'une quelconque des revendications 1 à 4, **caractérisée en ce que** l'agent de conditionnement est choisi parmi les esters de dimère d'acide gras et de polyéthylèneglycol de formule V donnée ci-dessous : 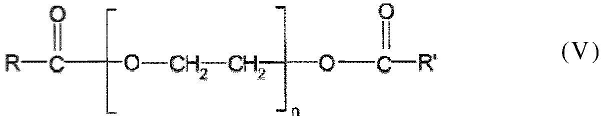 dans laquelle
- R et R', identiques ou différentes, représentent chacun un groupe alkyle ou alcényle, linéaire ou ramifié, comportant de 7 à 29 atomes de carbone, de préférence de 11 à 23 atomes de carbone, plus préférentiellement de 15 à 19 atomes de carbone, et mieux encore 17 atomes de carbone, et
- n est un nombre entier valant de 2 à 50, de préférence de 2 à 20 et mieux encore de 2 à 10.

8. Composition selon l'une quelconque des revendications précédentes, **caractérisée en ce que** l'agent de conditionnement est choisi parmi la vaseline liquide, l'huile d'olive, l'huile d'argan, l'huile d'avocat, l'huile d'amande d'abricot, l'huile de colza, l'huile de jojoba, l'huile de soja, l'huile de tournesol, et le distéarate de polyéthylèneglycol comportant de 2 à 50, de préférence de 2 à 20, plus préférentiellement de 2 à 10 et mieux encore de 2 à 5 motifs oxy-éthylène.

9. Composition selon l'une quelconque des revendications précédentes, **caractérisée en ce que** la proportion totale du ou des agent(s) de conditionnement présent(s) dans la composition finale est comprise entre 0,1 et 10 % en poids, et mieux encore entre 0,5 et 8 % en poids, par rapport au poids total de la composition.

10. Composition selon l'une quelconque des revendications précédentes, **caractérisée en ce qu'**elle comprend également un ou plusieurs tensioactif(s) choisi(s) parmi les tensioactifs anioniques, les tensioactifs amphotères ou zwittérioniques, les tensioactifs non-ioniques et les tensioactifs cationiques.

11. Composition selon la revendication précédente, **caractérisée en ce qu'**elle comprend un ou plusieurs tensioactif(s) anionique(s) en une proportion allant de 1 à 25 % en poids, et de préférence de 3 à 20 % en poids, par rapport au poids total de la composition, de préférence choisi(s) parmi les alkyl-sulfates, les alkyl-éther-sulfates, les alkyl-amido-éther-sulfates, les alkyl-sulfonates, les alkyl-amido-sulfonates, les acides alkyl-éther-carboxyliques, les N-(alkyle en C₁-C₄)-N-acyltaurates dont le groupe acyle comporte de 12 à 20 atomes de carbone, en particulier sous forme de sels de métaux alcalins ou alcalino-terreux, d'ammo-nium, d'amine ou d'amino-alcool, et leurs mélanges, et mieux encore, choisi(s) parmi les N-(alkyle en C₁-C₄)-N-acyl-taurates dont le groupe acyle comporte de 12 à 20 atomes de carbone, sous forme de sels de métaux alcalins ou alcalino-terreux.

12. Composition selon la revendication 10 ou 11, **caractérisée en ce qu'**elle comprend un ou plusieurs tensioactif(s) amphotère(s) ou zwittérionique(s) en une proportion allant de 0,1 à 15 % en poids, de préférence de 0,5 à 10 % en poids et mieux encore de 1 à 8 % en poids, par rapport au poids total de la composition, et de préférence choisi(s) parmi les (alkyle en C₈₋₂₀)-bétaïnes, les (alkyle en C₈₋₂₀)-amido-(alkyle en C₃₋₈)-bétaïnes et leurs mélanges.

13. Procédé de traitement cosmétique des matières kératiniques, notamment des fibres kératiniques humaines telles que les cheveux, **caractérisé en ce qu'**il consiste à appliquer sur lesdites matières kératiniques une composition selon l'une quelconque des revendications 1 à 12, puis à effectuer, en option, un rinçage après un éventuel temps de pause, en présence ou en l'absence de chaleur.

14. Utilisation d'une composition selon l'une quelconque des revendications 1 à 12, en tant que shampooing pour le nettoyage et le conditionnement des fibres kératiniques.

15. Utilisation selon la revendication 14, pour faciliter le coiffage et/ou améliorer la souplesse, le lissage et/ou le démêlage des cheveux.
